# EUROPÄISCHE PATENTSCHRIFT

(11) **EP 1 569 881 B1**
(45) Veröffentlichungstag und Bekanntmachung des Hinweises auf die Patenterteilung: **09.12.2009**
(21) Anmeldenummer: 03758074.3
(22) Anmeldetag: 28.10.2003
(51) Int. Cl.: C07C 1/20

(54) **VERFAHREN ZUR HERSTELLUNG VON 1-OLEFINEN DURCH KATALYTISCHE SPALTUNG VON 1-ALKOXYALKANEN**
METHOD FOR PRODUCING 1-OLEFINS BY CATALYTICALLY SPLITTING 1-ALKOXYALKANES
PROCEDE DE PRODUCTION DE 1-OLEFINES PAR SEPARATION CATALYTIQUE DE 1-ALCOXYALCANES

(30) Priorität: 10.12.2002 DE 10257499
(43) Veröffentlichungstag der Anmeldung: 07.09.2005
(73) Patentinhaber: Evonik Oxeno GmbH, 45772 Marl (DE)
(72) Erfinder: KAIZIK, Alfred, 45772 Marl (DE); MASCHMEYER, Dietrich, 45657 Recklinghausen (DE); RÖTTGER, Dirk, 45657 Recklinghausen (DE); NIERLICH, Franz, 45768 Marl (DE); BORGMANN, Cornelia, 60486 Frankfurt (DE)
(74) Vertreter: Hirsch, Hans-Ludwig
(86) Internationale Anmeldenummer: PCT/EP2003/011919
(87) Internationale Veröffentlichungsnummer: WO 2004/052809

(56) Entgegenhaltungen:
- DE-A- 10 105 751
- GB-A- 673 547
- GB-A- 2 128 972
- US-A- 4 395 580

## Beschreibung

Die vorliegende Erfindung betrifft ein Verfahren zur Herstellung von 1-Olefinen aus 1-Alkoxyalkanen, insbesondere die Herstellung von 1-Octen aus 1-Alkoxyoctan, durch katalytische Alkoholabspaltung unter nicht isomerisierenden Bedingungen.

Olefine zählen wegen ihrer Reaktivität zu den wichtigsten Synthesebausteinen der organischen Chemie. Sie sind Vorstufen für eine Vielzahl von Verbindungen, wie beispielsweise Aldehyde, Ketone, Alkohole, Karbonsäuren und Halogenverbindungen. In großen Mengen werden sie zur Herstellung von Homo- oder Cooligomeren und Homo- und Copolymeren verwendet, wie beispielweise Polyethylen oder Polypropylen.

Ethen und Propen werden durch Steamcracking oder durch katalytische Spaltung von Kohlenwasserstoffen weltweit in großen Mengen hergestellt. Dabei fallen beträchtliche Mengen an C₄-Olefinen (Isobuten, 1-Buten, 2-Butene) und C₅-Olefine an.

Olefine mit mehr als vier C-Atomen nehmen in ihrer Isomerenzahl rasch zu. Eine Trennung solcher Isomerengemische, wie sie z.B. bei Crackverfahren anfallen, ist technisch aufwendig.

Höhere Olefine können linear oder verzweigt sein, wobei die Lage der Doppelbindung endständig (terminal, α-Olefine, 1-Olefine) oder innenständig (intern) sein kann. Die linearen α-Olefine (LAO) stellen davon die industriell bedeutendste Produktgruppe dar.

Geradkettige α-Olefine wie 1-Hexen und 1-Octen werden in großen Mengen in der Produktion verschiedener chemischer Produkte eingesetzt. So werden beispielweise aus 1-Octen oberflächenaktive Stoffe, Weichmacher, Schmierstoffe und Polymere hergestellt. Ein wirtschaftlich wichtiges Einsatzgebiet ist die Verwendung von 1-Octen als Comonomer in Polymerkunstoffen, insbesondere in modifiziertem Polyethylen und modifiziertem Polypropylen.

Höhere lineare Olefine werden z. B. durch Aufbaureaktionen auf der Basis von Ethen bzw. durch Dehydrochlorierung von n-Chlorparaffinen erzeugt.

Ethen kann mit Hilfe von Ziegler-Katalysatoren (Triethylaluminium) oligomerisiert werden, wobei ein Gemisch von unverzweigten α-Olefinen mit gerader C-Zahl anfällt.
Weitere Herstellverfahren für α-Olefine basieren ebenfalls auf Ethen als Einsatzstoff, unterscheiden sich jedoch wesentlich durch den zur Oligomerisierung eingesetzten Katalysator (siehe: "Applied Homogeneous Catalysis with Organometallic Compounds", Edited by B.Comils,W.A.Herrmann, VCH Verlag Weinheim 1996, Vol. 1, S. 245-258). So wird in dem von Shell entwickelten SHOP-Prozess (Shell higher olefin process) ein Nickel-Phosphinkomplex-Katalysator für die Ethen-Oligomerisierung verwendet (siehe: K.Weissermel, H.-J.Arpe, "Industrielle Organische Chemie", VCH Verlag Weinheim 1994, 4.Aufl, S. 95 ff.).
Nach einer Variante des SHOP-Prozesses können aus Ethen unverzweigte α-Olefin mit gerader und ungerader C-Zahl hergestellt werden. Dieses Verfahren umfasst drei Reaktionsschritte, nämlich Ethenoligomerisierung, Doppelbindungs-Isomerisierung, d. h. Verschiebung der Doppelbindungen, und Kreuzmetathese (Ethenolyse) des Olefingemisches mit innenständigen Doppelbindungen mit Ethen.

Für die Herstellung von Olefinen auf Basis von n-Paraffinen haben sich unter anderem thermisches Cracken, katalytische Dehydrierung und chlorierende Dehydrierung (Chlorierung und anschließende Chlorwasserstoffabspaltung) bewährt.

Bei diesen Verfahren entstehen Olefine mit überwiegend innenständigen Doppelbindungen, die durch Kreuzmetathese zu α-Olefinen umgesetzt werden können.

Die obengenannten Verfahren zur Herstellung von höheren Olefinen haben allerdings den Nachteil, dass immer eine Vielzahl an α-Olefinen unterschiedlicher Kettenlänge gebildet wird, die zum einen aufwendig getrennt werden müssen, zum anderen die Ausbeute des gewünschten α-Olefins stark mindern.

Die zur Zeit für die Herstellung von 1-Octen angewandten Verfahren basieren hauptsächlich auf dem Rohstoff Ethen. Man erhält Olefingemische, aus denen 1-Octen durch Destillation gewonnen wird. Beispielsweise kann nach dem SHOP-Verfahren unter optimierten Reaktionsbedingungen nur ein Olefingemisch mit einem 1-Octen-Gehalt von maximal 25 Gew.-% erhalten werden.

Neben den Verfahren auf Ethen-Basis hat darüber hinaus auch die Isolierung von 1-Octen aus dem Produktspektrum der Fischer-Tropsch-Synthese eine technische Bedeutung erlangt.

In der Literatur sind neben den Herstellverfahren auf Basis von Ethen auch Verfahren bekannt, die als Rohstoff für die 1-Octen-Herstellung 1,3-Butadien verwenden.

Bei der Verwendung von 1,3-Butadien als Rohstoffbasis wird 1-Octen nicht auf dem direkten Syntheseweg, beispielsweise über eine Dimerisierung erhalten, sondern über mehrere Reaktionsschritte. So beschreibt WO 92/10450 ein Verfahren, bei dem 1,3-Butadien vorzugsweise mit Methanol oder Ethanol zu einem 2,7-Octadienylether umgesetzt wird, der nach Hydrierung zum Octylether (z. B. 1-Methoxyoctan) an einem sauren-γ-Al₂O₃ zum 1-Octen gespalten wird. In EP 0 440 995 wird ein analoger Weg beschritten, die Umsetzung erfolgt im ersten Schritt jedoch mit einer Carbonsäure. Charakteristisch für alle Verfahren ist der erste Prozessschritt, den man allgemein als Telomerisation bezeichnet. Bei der Telomerisation wird allgemein ein Telogen (wie z.B. Wasser, Methanol, Ethanol und Carbonsäure) mit einem Taxogen (1,3-Butadien, 2 Äquivalente) zu einem Telomer umgesetzt.

Bei den bekannten Verfahren zur Herstellung von 1-Octen auf der Basis von Butadien, wie beispielweise in WO 92/10450 oder EP 0 440 995 beschrieben, wird das 1-Octen durch Spaltung eines an 1-Position substituierten n-Octans (Alkoxyoctan) erhalten. Die Selektivitäten in diesem Schritt sind dabei oftmals unbefriedigend. So wird in WO 92/10450 bei der Spaltung von 1-Methoxyoctan an reinen Aluminiumoxid oder an sauer modifizierten Aluminiumoxid bei einem Umsatz von 80 % eine Selektivität zu Octenen von nur 66 % erreicht.
Auch die Spaltung von 1- und 2-Octanolen und C₈-Alkylestern sowie 1-Alkoxyoctan zu 1-Octen ist in der Patentliteratur bekannt.

Die Spaltung von tert.-Butanol zu Wasser und Isobuten wird in EP 0 726 241 mittels saurer Ionenaustauscher in einer Reaktivdestillation durchgeführt. Die Übertragung dieses Reaktorkonzepts zur Spaltung von Methyl-tert.-butylether zu Wasser und Isobuten kann EP 1 149 814 entnommen werden.

JP 02172924 beschreibt die Spaltung (Dehydratation) von 1-Octanol, das durch eine Telomerisationsreaktion von 1,3-Butadien mit Wasser und anschließende Hydrierung erhalten wurde zu 1-Octen und Wasser. Als Katalysator für die Spaltung kommt unter anderem ein mit Natriumhydroxid modifiziertes Calciumphosphat zum Einsatz.

EP 0 440 995 beschreibt die thermische Spaltung von Alkylestern, erhalten aus einer Telomerisationsreaktion und anschließender Hydrierung, zu 1-Octen. Es werden keine Katalysatoren in der Spaltungsreaktion eingesetzt.

Die Spaltung von Alkoxyalkanen (Ether) zu Olefinen ist ebenfalls bekannt. Einige Arbeiten wurden zu Beginn des 20. Jahrhunderts publiziert, beispielsweise die Spaltung von Ethern über saurem Ton (japanese acid clay) (W. Ipatiew, Berichte der Deutschen Chemischen Gesellschaft, 1904, 37,2961; K. Kashima, Bull. Chem. Soc. Jpn. 1930, 25).

Die Spaltung eines Methylethers in Gegenwart von Aluminiumoxid, Aluminiumphosphaten, Aluminiumsilikaten und Mischungen von Aluminiumsilikaten mit Metallphosphaten und Metallsulfaten ist Gegenstand des Patents US 2 561 483.

In CN 1158277 A werden Katalysatoren, ausgewählt aus modifiziertem Siliziumoxid (SiO₂), Thoriumoxid (ThO₂), den Oxiden der Erdalkalimetalle, der seltenen Erden und der Metalle der Gruppe IV B für die Spaltung von Alkoxyalkanen (Ethern) beansprucht.

CN 1165053 offenbart die Spaltung von 1-Methoxyoctan (Octylmethylether) zu 1-Octen in Gegenwart eines mit Magnesiumoxid modifizierten Siliziumoxides. Mit diesen MgO-SiO₂-Katalysatoren konnten bei 1-Methoxyoctan-Umsätzen von über 80 % 1-Octen -Selektivitäten von über 95 % erzielt werden.

Die Spaltung von 1- Alkoxyalkanen kann sowohl in der Flüssigphase als auch in der Gasphase durchgeführt werden. In allgemeinen wird die Spaltung von 1-Alkoxyalkanen zu 1-Olefinen als eine heterogen katalysierte Gasphasenreaktion durchgeführt. DE 101 05 751 offenbart zu diesem Zweck die Verwendung von SiO₂ und Al₂O₃, beides unmodifiziert als Reinstoff.

Zusammenfassend kann gesagt werden, dass die bekannten Verfahren zur Spaltung von Alkoxyalkanen oder Alkanolen an sauren Katalyatoren wie sulfonierten Ionenaustauscherharzen, Al₂O₃ oder SiO₂ durchgeführt werden. Saure Verbindungen katalysieren jedoch nicht nur die Spaltung, sondern auch die Isomerisierung der erhaltenen 1-Olefine zu Olefinen mit innenbeständigen Doppelbindungen.

Neben dem gewünschten Wertprodukt 1-Olefin werden daher als Nebenprodukte unerwünschte innenständige Olefine gebildet, die nur schlecht vom Wertprodukt 1-Olefin abgetrennt werden können. Unter isomerisierenden Bedingungen kann ein 1-Olefin bis zur Einstellung des thermodynamischen Gleichgewichts in Olefine mit innenständigen Doppelbindungen umgesetzt werden. Bei der Herstellung von 1-Olefinen ist die Bildung von Olefinen mit innenständigen Doppelbindungen (interne Olefinisomere) aus zwei Gründen unerwünscht, zum einen wegen des Ausbeuteverlustes und zum anderen wegen des technischen Aufwands für die Abtrennung des 1-Olefins von internen Olefinisomeren, da die Siedepunkte der isomeren Olefine nahe beieinander liegen.

Die Forderung für ein technisches Verfahren zur Herstellung eines 1-Olefins aus einem 1-Alkoxyalkan oder aus einem Alkanol ist daher eine selektive Spaltung zu dem Zielprodukt, unter weitgehender Minimierung einer anschließenden Isomerisierung des gebildeten 1-Olefins.

Es wurde nun gefunden, dass in Gegenwart von basischen Katalysatoren auf Basis von modifizierten Aluminiumoxiden oder Zirkondioxid im Vergleich zu den bislang verwendeten saueren Katalysatoren die Selektivität der Spaltung von Methoxyalkanen zu 1-Olefinen und das Verhältnis von 1-Olefin zu innenständigen Olefinen deutlich verbessert wird.

Gegenstand der Erfindung ist ein Verfahren zur Herstellung von α-Olefinen mit 3 bis 16 Kohlenstoffatomen durch katalytische Spaltung von 1-Alkoxyalkanen, wobei die Spaltung an Aluminiumoxid und/oder Zirkoniumdioxid enthaltend 0.01 bis 10 Gew.-% mindestens eines

Alkali- und/oder Erdalkalioxids, durchgeführt wird.

Im erfindungsgemäßen Verfahren können Alkoxyalkane mit der allgemeinen Struktur (R1)-CH₂-CH₂-O(R2) an den erfindungsgemäßen Katalysatoren zu den entsprechenden 1-Olefinen mit der allgemeinen Struktur (R1)-CH=CH₂ umgesetzt werden.
Analog können Verbindungen der allgemeinen Formeln (R1)-CH₂CH₂-OH oder (R1)CH(OH)CH₃ zu 1-Olefinen mit der Formel (R1)-CH = CH₂ umgesetzt werden.

Die Gruppe (R1) ist dabei bevorzugt eine Kohlenwasserstoffgruppe mit 1 bis 14 Kohlenstoffatomen, die Gruppe (R2) bevorzugt eine Kohlenwasserstoffgruppe mit 1 bis 4 Kohlenstoffatomen

Bevorzugte Produkte des erfindungsgemäßen Verfahrens sind 1-Octen, 1-Penten, Isobuten oder 1-Buten.

Die mit dem erfindungsgemäßen Verfahren hergestellten α-Olefine weisen bevorzugt 4 bis 8 Kohlenstoffatome auf.

Bei der Spaltung von 1-Alkoxyalkanen werden insbesondere 1-Methoxyoctan, 1-Ethoxyoctan, tert.-Butylmethylether und/oder tert.-Amylmethylether, tert.-Amylethylether oder tert.-Amylbutylether zu den entsprechenden α-Olefinen und Alkoholen gespalten, wobei als Olefin bevorzugt 1-Octen, 1-Penten, Isobuten oder 2-Methyl-1-Buten erhalten wird.

Die Spaltung der 1-Alkoxyalkane zum 1-Olefin wird im erfindungsgemäßen Verfahren bevorzugt als eine heterogen katalysierte Gasphasenreaktion durchgeführt.

Als Katalysatoren bei dem erfindungsgemäßen Verfahren werden bevorzugt basische und stark basische Katalysatoren verwendet. Die erfindungsgemäß eingesetzten Katalysatoren enthalten als Hauptkomponenten Aluminiumoxid und/oder Zirkoniumdioxid sowie Alkalimetall- und/oder Erdalkalimetalloxide. Als weitere Komponenten können im Katalysator Titandioxid, Siliciumdioxid und/oder Thoriumoxid mit 0,01 bis 3 Gew.-%, bevorzugt 0,5 bis 5 Gew.-% enthalten sein. Diese Katalysatoren sind basisch im Sinne der vorliegenden Erfindung.

Der Anteil an basischen Metalloxiden (Hydroxide werden in Oxide umgerechnet) im Katalysator beträgt bevorzugt 0,01 bis 10 Massen-%, besonders bevorzugt 0,1 bis 5 Massen-%, insbesondere bevorzugt 0,1 bis 3 Massen-%. Bevorzugte Alkalimetalloxide sind Natrium- und/oder Kaliumoxid. Als Erdalkalimetalloxide werden bevorzugt Magnesium-, Strontium- und/oder Bariumoxid eingesetzt.

Bevorzugt werden γ-Aluminiumoxide mit einer BET-Oberfläche von 80 bis 350 m²/g, bevorzugt 120 - 250 m²/g eingesetzt. Die Katalysatoren werden nach bekannten Methoden hergestellt. Gängige Methoden sind beispielsweise Fällung, Tränkung oder Besprühung eines Al₂O₃-Körpers mit einer entsprechenden Salzlösung und anschließende Calcinierung.

Die Katalysatoren werden zweckmäßig in Form von Kugeln, Tabletten, Zylindern, Strangextrudaten oder Ringen eingesetzt.

Die Spaltung des 1-Alkoxyalkan kann in Gegenwart von Stoffen erfolgen, die unter den Spaltbedingungen inert beziehungsweise weitgehend inert sind. So können beispielsweise Stickstoff oder Argon, aber auch Wasser, Wasserdampf oder Alkane wie beispielsweise Methan, Propan oder auch Dimethylether zugesetzt werden. Bevorzugt liegt der Anteil dieser inerten Stoffe zwischen 0 und 90 Vol.-%, besonders bevorzugt zwischen 0 und 50 und, zwischen 0 und 30 und, zwischen 0 und 20 oder zwischen 0 und 10 Vol.-%.
Im erfindungsgemäßen Verfahren wird die Spaltung der 1-Alkoxyalkane in der Gas- oder Flüssig/Gas-Mischphase an suspendierten oder stückigen im Festbett angeordneten Katalysatoren kontinuierlich oder diskontinuierlich durchgeführt. Bevorzugt wird die kontinuierliche Spaltung an im Festbett angeordneten Katalysator durchgeführt.

Bei der kontinuierlichen Spaltung können unterschiedliche Verfahrensvarianten gewählt werden. Sie kann adiabatisch, polytrop vorzugsweise praktisch isotherm, d. h. mit einer Temperaturdifferenz von typischerweise kleiner als 10 °C, ein- oder mehrstufig durchgeführt werden. Im letzteren Falle kann man alle Reaktoren, zweckmäßig Rohrreaktoren praktisch isotherm betreiben. Bevorzugt wird die Spaltung im geraden Durchgang betrieben. Sie kann jedoch auch unter Produktrückführung betrieben werden. Es ist möglich, zwischen den Reaktoren zumindest teilweise die Produkte abzutrennen.

Die Spaltung von 1-Alkoxyalkanen kann bei Temperaturen zwischen 100 und 600 °C, bevorzugt zwischen 200 und 450 °C, besonders bevorzugt zwischen 280 und 350 °C erfolgen. Die Spaltung kann auch bei deutlich geringeren Temperaturen wie z. B. 100 - 250 °C, bevorzugt 100 - 200 °C durchgeführt werden, wie z. B. bei der Spaltung von Methyl-tert.-butylether (MTBE) oder tert.-Amylmethylether (TAME).

Der Druck (absolut), unter dem die Spaltung durchgeführt wird, liegt typischerweise zwischen 0,1 und 25 bar. Bevorzugt werden Drücke zwischen 0,2 und 10 bar, besonders bevorzugt zwischen 1 und 5 bar. Die Weight-Hourly-Space-Velocity (WHSV), angegeben in Gramm Edukt pro Gramm Katalysator pro Stunde, beträgt bevorzugt 0,01 bis 30 h⁻¹, besonders bevorzugt 0,1 -15 h⁻¹, ganz besonders bevorzugt 0,5 - 10 h⁻¹.

Die Spaltung der 1-Alkoxyalkane zu 1-Olefinen kann unter vollständigem oder teilweisen Umsatz durchgeführt werden. Nicht umgesetztes Edukt kann, nach Abtrennung des gebildeten 1-Olefins und gegebenenfalls anderer Spaltungsprodukte, in die Spaltung zurückgeführt werden. Es ist dabei auch möglich, nur das 1-Olefin und gegebenenfalls einen Teil der Spaltprodukte abzutrennen und den Rest in die Vorreinigung vor der eigentlichen Spaltung zurückzuführen.

Bevorzugt wird die Spaltung unter teilweisem Umsatz durchgeführt. Der Umsatz beträgt dabei zwischen 10 und 95 %, besonders bevorzugt zwischen 30 und 95 %, ganz besonders bevorzugt zwischen 70 und 95 %.

Die Abtrennung des Zielprodukts, des 1-Olefins, von den anderen Komponenten des Austrags der Spaltung erfolgt nach bekannten Verfahren wie beispielsweise Phasenseparation, Extraktion, Wäsche oder Destillation.
Der Reaktionsaustrag wird in eine Olefinfraktion und eine Fraktion, die nicht umgesetztes Alkoxyalkan, Alkohol, Wasser und gegebenenfalls andere Nebenprodukten enthält, getrennt. Die Olefinfraktion besteht zu mehr als 85 %, bevorzugt mehr als 90, insbesondere 95 bis über 98 % aus 1-Olefin. Optional wird sie zu noch reinerem 1-Olefin aufgearbeitet. Das nicht umgesetzte Edukt kann in den Spaltungsreaktor zurückgeführt werden.

Die nach dem erfindungsgemäßen Verfahren hergestellten 1-Olefine können als Comonomere bei der Herstellung von Polyolefinen verwendet werden. Weiterhin können sie Ausgangsstoff für organische Synthesen sein.

Das erfindungsgemäße Verfahren hat folgende Vorteile: Im Produktgemisch nach der Spaltung ist der Anteil an innenständigen Olefinen, die nicht bzw. nur schwer vom dem Wertprodukt 1-Olefin abtrennbar sind, gering. Die Olefinfraktion bei der Spaltung von 1-Methoxyoctan besteht zu 91,5 bis 98,5 % aus 1-Octen, 1 bis 8,4 % aus 2-Octen und 0,1 bis 2,0 % aus 3-, 4-Octenisomeren, sodass eine wirtschaftliche Gewinnung von reinem 1-Octen möglich ist.

Die eingesetzten Katalysatoren besitzen im Prozess eine lange Lebensdauer, da eine Verkokung wie sie beim Einsatz von sauren Katalysatoren wie SiO₂ beobachtet wird, nahezu vollständig unterbleibt.

Die folgenden Beispiele sollen die Erfindung beschreiben, ohne ihre Anwendungsbreite einzuschränken, die sich aus der Beschreibung und den Patentansprüchen ergibt:

### Beispiele

### Beispiel 1: Herstellung eines Ba-modifzierten Al₂O₃-Katalysators

Für die Herstellung des erfindungsgemäßen Katalysators wurde ein saures γ-Aluminiumoxid mit Na₂O-Gehalt < 300 ppm der Fa.Axens verwendet. Das Aluminiumoxid mit einer BET-Oberfläche von 225 m²/g und einem Porenvolumen von 0,68 ml/g lag als Extrudat ( Zylinder mit einer Länge von 4-6 mm und einem Durchmesser von 1,25 mm) vor. Als Barium-Vorläufer für die basische Modifizierung des Aluminiumoxides mit Bariumoxid (BaO) wurde Bariumnitrat Ba(NO₃)₂ eingesetzt.
Vor der Aufbringung des Bariumssalzes wurde das Aluminiumoxid zuerst bei 90 °C 5 Stunden lang in einem Umluftrockenschrank getrocknet. Die getrockneten Strangextrudate wurden anschließend in einer Rotationstrommel (Drageetrommel) bei Raumtemperatur mit der Bariumnitrat-Lösung über eine Sprühdüse imprägniert.
Der gewünschte Barium-Gehalt in den zu imprägnierenden Strangextrudaten kann über die Konzentration der Ba-Salzlösung eingestellt werden. Nach der Imprägnierung wurden die mit dem Barium-Salz beladenen Al₂O₃-Extrudate zuerst bei 110 °C 5 Stunden lang in einem Umluftrockenschrank getrocknet. Die anschließende Kalzinierung, bei der das Bariumsalz zu Bariumoxid bzw. einer Barium/Aluminium/Sauerstoffverbindung umgewandelt wird, erfolgte in einem Wirbelbettreaktor in Luftstrom 10 Stunden lang bei 450 °C.

### Beispiel 2: Herstellung eines Na-modifzierten Al₂O₃-Katalysators

Für die Herstellung des mit Na-modifzierten Al₂O₃-Katalysators wurde das im Beispiel 1 beschriebene saure γ-Aluminiumoxid mit Na2O-Gehalt < 300 ppm der Fa.Axens verwendet. Als Imprägnierlösung wurde eine wässrige Natronlauge verwendet.
Die Aufbringung der Natronlauge auf die Al₂O₃-Strangetrudate und die thermische Nachbehandlung (Trocknung und Kalzinierung) des Katalysators wurden nach dem in Beispiel 1 beschriebenen Herstellungsgang durchgeführt.

### Beispiel 3: (Vergleichsbeispiel) Spaltung von 1-Methoxyoctan an einem unmodifizierten γ-Al₂O₃- Katalysator

1-Methoxyoctan (1-MOAN, Methyl-n-octylether), erhalten durch Hydrierung von 1-Methoxyoctadien (Telomerisierungsprodukt von 1,3-Butadien mit Methanol), wurde mit einer Reinheit von rd. 98 Gew.-% (2 % Hochsieder) für die Spaltung in einem elektrisch beheizten Durchfluss-Festbettreaktor in Gegenwart eines Katalysators eingesetzt. Bei dem Katalysator (Kat.1) handelte es sich um ein handelsübliches, oberflächenreiches saures γ-Al₂O₃ (BET-Oberfläche 225 m²/g, Porenvolumen 0,68 cm³/g) mit der Bezeichnung Spheralite 521C der Fa. Axens.

Vor dem Eintritt in den Reaktor wurde das flüssige Edukt in einem vorgeschalteten Verdampfer bei 220 °C verdampft. Bei einer Reaktionstemperatur von 300 °C und einem Druck von 1 bar im Reaktor wurden stündlich 75,0 g/h Edukt durch 13,9 g Katalysator in Extrudatenform in der Gasphase, entsprechend einem WHSV-Wert von 5,4 h⁻¹, durchgeleitet. Das gasförmige Produkt wurde in einem Kühler abgekühlt und in flüssiger Form in einer Glasvorlage gesammelt.

Die GC-Analyse des Spaltungsproduktes ist in Tabelle 1, Spalte 2, wiedergegeben.
Nach den vorliegenden Ergebnissen wurden bei einem 1-MOAN-Umsatz von rd. 84,6 % folgende Octen-Selektivitäten erzielt : Wertprodukt 1-Octen Sel. 86,7 % , Nebenprodukte, innenständige C₈-Isomere : 2-Octene Sel. 5,7 % und 3 /4-Octene Sel. 2,1 %

**Tabelle 1: Spaltung von 1-Methoxyoctan am unmodifzierten γ-Al₂O₃- Katalysator**

| | Beispiel 3 | Beispiel 4 | Beispiel 5 |
|---|---|---|---|
| Komponente | Kat.1 (Vergleich) | Kat.2 (Erfindung) | Kat.3 (Erfindung) |
| 1-Octen | 56,59 | 59,39 | 60,41 |
| t-4-Octen | 0,38 | 0,01 | 0,01 |
| 3-Octene/c-4-Octen | 0,96 | 0,44 | 0,33 |
| t-2-Octen | 0,96 | 0,99 | 0,73 |
| c-2-Octen | 2,75 | 2,97 | 1,75 |
| Methanol | 2,45 | 2,48 | 2,90 |
| Dimethylether | 11,26 | 10,89 | 10,84 |
| Wasser | 4,40 | 4,26 | 4,24 |
| 1-MOAN | 16,38 | 15,84 | 13,16 |
| Rest | 3,88 | 2,74 | 5,71 |

### Beispiel 4: (gemäß der Erfindung) Spaltung am mit Ba-modifizierten γ-Al₂O₃-Katalysator

Das Produkt der Hydrierung von 1-Methoxyoctadien das 1-Methoxyoctan (1-MOAN) wurde mit einer Reinheit von rd. 98 Gew.-% (2 % Hochsieder) für die Spaltung in einem Durchfluss-Festbettreaktor, wie in Beispiel 3 beschrieben, in Gegenwart eines mit BaO modifizierten Aluminiumoxids (Al₂O₃ mit 1,0 Gew.-% BaO) aus dem Beispiel 1 eingesetzt.
Bei einer Reaktionstemperatur von 300 °C und einem Druck von 1 bar im Reaktor wurden stündlich 50 g Methoxyoctan durch 14,1 g Katalysator in Zylinderform in der Gasphase, entsprechend einem WHSV-Wert von 3,5 h⁻¹, durchgeleitet. Wie im Beispiel 3, wurde das gasförmige Produkt in einem Kühler abgekühlt und in flüssiger Form in einer Glasvorlage gesammelt.
Die GC-Analyse des Spaltungsproduktes ist in Tabelle 1, Spalte 3 (Kat.2) , wiedergegeben.
Wie man aus der Tabelle 1 entnehmen kann, wird das 1-MOAN an mit Ba-modifiziertem Aluminiumoxid im Vergleich zum unmodifizierten, sauren γ-Aluminiumoxid (Kat.1) bei vergleichbaren MOAN-Umsätzen deutlich selektiver zum Wertprodukt 1-Octen bei geringerer Bildung von 3- und 4-Octenisomeren gespalten.
Bei einem 1-MOAN-Umsatz von rd. 83,7 % wurden an dem erfindungsgemäßen Katalysator folgende Octen-Selektivitäten erzielt : Wertprodukt 1-Octen Sel. 94,2 %, Nebenprodukte innenständige C₈-Isomere: 2-Octene Sel. 5,0 % und 3 /4-Octene Sel. 0,7 %.

### Beispiel 5: (gemäß der Erfindung) Spaltung am mit Na-modifizierten γ-Al₂O₃ -Katalysator

Wie in den Beispielen 3 und 4 wurde das Produkt der Hydrierung von 1-Methoxyoctadien das 1-Methoxyoctan (1-MOAN, Methyl-n-octylether) als Edukt für die Gasphasen-Spaltung in einem Durchfluss-Festbettreaktor verwendet. Als Katalysator wurde ein mit Natronlauge modifiziertes Aluminiumoxid (Al₂O₃ mit 1,5 Gew.-% Na₂O) aus dem Beispiel 2 eingesetzt. Bei einer Reaktionstemperatur von 350 °C im Reaktor wurden stündlich 25 g Methoxyoctan durch 13,5 g Katalysator in Extrudatenform in der Gasphase, entsprechend einem WHSV-Wert von 1,8 h⁻¹, durchgeleitet. Das gasförmige Produkt wurde in einem Kühler abgekühlt und in flüssiger Form in einer Glasvorlage gesammelt.
Die GC-Analyse des Spaltungsproduktes ist in Tabelle 1, Spalte 4, wiedergegeben.
Wie man aus der Tabelle 1 entnehmen kann, wird das 1-MOAN auch an einem mit Na-modifizierten γ-Aluminiumoxid (Kat. 3) bei geringer Bildung von 3-, 4-Octenen mit einer hohen 1-Octen-Selektivität zum gewünschten Wertprodukt 1-Octen gespalten.
Bei einem 1-MOAN-Umsatz von rd. 86,2 % wurden folgende Octen-Selektivitäten erzielt: Wertprodukt 1-Octen Sel. 93,8 % , Nebenprodukte innenständige C₈-Isomere : 2-Octene Sel. 3,9 % und 3 /4-Octene Sel. 0,5 %.

Die unter Rest aufgeführten Nebenprodukte enthalten Komponenten, die ebenfalls zum 1-Octen gespalten werden können, unter anderem Dioctylether. Auch diese können ggf. in die Spaltung zurückgeführt werden.

### Beispiel 6 : (gemäß der Erfindung) Spaltung von Methyl-tert.-butylether (MTBE) an einem mit Na-modifizierten γ-Al₂O₃

Methyl-tert.-butylether (MTBE, ter.-Butyl-methylether) von Oxeno mit einer Reinheit von 99,94 Gew.-% wurde als Edukt für die katalytische Gasphasen-Spaltung in einem Durchfluß-Festtbettreaktor eingesetzt. Als Katalysator für die Spaltung wurde ein mit Natronlauge modifiziertes γ-Aluminiumoxid (Al₂O₃ mit 1,5 Gew.-% Na₂O) aus dem Beispiel 2 verwendet.

Vor dem Eintritt in den Reaktor wurde das flüssige Edukt in einem vorgeschalteten Verdampfer bei 180 °C verdampft. Bei einer Reaktionstemperatur von 235 °C und einem Druck von 1 bar wurden stündlich 15 g Methyl-tert.-butylether durch 20,0 g Katalysator in Extrudatenform in der Gasphase, entsprechend einem WHSV-Wert von 0,75 h⁻¹, durchgeleitet. Der gasförmige Reaktionsaustrag wurde in einem Kühler abgekühlt und in flüssiger Form in einer Glasvorlage gesammelt.
Nach der GC-Analyse enthält der Reaktionsaustrag neben dem nicht umgesetzten Edukt Methyl-tert.-butylether (38,0 Gew.-% MTBE) folgende Spaltprodukte:
38,83 Gew.-% Isobuten, 21,58 Gew.-% Methanol, 1,06 Gew.-% Dimethylether, 0,43 Gew.-% Wasser und 0,10 Gew.-% 2,4,4-Trimethylpentene.
Nach diesem Ergebnis werden bei MTBE-Umsätzen von rd. 62% sehr hohe Selektivitäten (> 99,7 %) zu dem Zielprodukt Isobuten erzielt. Die Selektivitäten der MTBE-Spaltung zu Methanol liegen, bedingt durch die Dimethylether-Bildung bei rd. 95,5 %.

## Patentansprüche

1. Verfahren zur Herstellung von α-Olefinen mit 3 bis 16 Kohlenstoffatomen durch katalytische Spaltung von 1-Alkoxyalkanen,
**dadurch gekennzeichnet,**
**dass** die Spaltung an Aluminiumoxid und/oder Zirkoniumdioxid enthaltend 0.01 bis 10 Gew.-% mindestens eines Alkali- und/oder Erdalkalioxids, durchgeführt wird.

2. Verfahren nach Anspruch 1,
**dadurch gekennzeichnet,**
**dass** als Alkalioxid Kalium- und/oder Natriumoxid eingesetzt wird.

3. Verfahren nach Anspruch 1 oder 2,
**dadurch gekennzeichnet,**
**dass** als Erdalkalioxid Strontium-, Magnesium- und/oder Bariumoxid eingesetzt wird.

4. Verfahren nach Anspruch 1 bis 3,
**dadurch gekennzeichnet,**
**dass** der Katalysator zusätzlich 0,01 bis 5 Gew.-% Titanoxid, Siliziumdioxid und/oder Thoriumoxid enthält.

5. Verfahren nach einem der Ansprüche 1 bis 4,
**dadurch gekennzeichnet,**
**dass** die katalytische Spaltung bis zu einem Umsatz von 10 bis 95 % durchgeführt wird.

6. Verfahren nach einem der Ansprüche 1 bis 5,
**dadurch gekennzeichnet,**
**dass** die Spaltung in der Gasphase durchgeführt wird.

7. Verfahren nach einem der Ansprüche 1 bis 6,
**dadurch gekennzeichnet,**
**dass** die Spaltung bei einer Temperatur von 100 bis 600 °C durchgeführt wird.

8. Verfahren nach einem der Ansprüche 1 bis 7,
**dadurch gekennzeichnet,**
**dass** 1-Methoxyoctan, 1-Ethoxyoctan, tert.-Butylmethylether, tert.-Amylmethylether, tert.-Amylethylether oder tert.-Amylbutylether zu den entsprechenden α-Olefinen und Alkoholen gespalten wird.

## Claims

1. Process for preparing α-olefins having from 3 to 16 carbon atoms by catalytically cleaving 1-alkoxyalkanes,
**characterized in that**
the cleavage is carried out over alumina and/or zirconia containing from 0.01 to 10% by weight of at least one alkali metal and/or alkaline earth metal oxide.

2. Process according to Claim 1,
**characterized in that**
the alkali metal oxide used is potassium oxide and/or sodium oxide.

3. Process according to Claim 1 or 2,
**characterized in that**
the alkaline earth metal oxide used is strontium oxide, magnesium oxide and/or barium oxide.

4. Process according to Claims 1 to 3,
**characterized in that**
the catalyst additionally contains from 0.01 to 5% by weight of titanium oxide, silicon dioxide and/or thorium oxide.

5. Process according to one of Claims 1 to 4,
**characterized in that**
the catalytic cleavage is carried out up to a conversion of from 10 to 95%.

6. Process according to one of Claims 1 to 5,
**characterized in that**
the cleavage is carried out in the gas phase.

7. Process according to one of Claims 1 to 6,
**characterized in that**
the cleavage is carried out at a temperature of from 100 to 600°C.

8. Process according to one of Claims 1 to 7,
**characterized in that**
1-methoxyoctane, 1-ethoxyoctane, tert-butyl methyl ether, tert-amyl methyl ether, tert-amyl ethyl ether or tert-amyl butyl ether is cleaved to the corresponding α-olefins and alcohols.

## Revendications

1. Procédé pour la préparation d'α-oléfines comprenant 3 à 16 atomes de carbone par dissociation catalytique de 1-alcoxyalcanes, **caractérisé en ce que** la dissociation est réalisée sur de l'oxyde d'aluminium et/ou du dioxyde de zirconium contenant 0,01 à 10% en poids d'au moins un oxyde de métal alcalin et/ou de métal alcalino-terreux.

2. Procédé selon la revendication 1, **caractérisé en ce qu'**on utilise comme oxyde de métal alcalin de l'oxyde de potassium et/ou de sodium.

3. Procédé selon la revendication 1 ou 2, **caractérisé en ce qu'**on utilise comme oxyde de métal alcalino-terreux de l'oxyde de strontium, de magnésium et/ou de baryum.

4. Procédé selon la revendication 1 à 3, **caractérisé en ce que** le catalyseur contient en outre 0,01 à 5% en poids d'oxyde de titane, de dioxyde de silicium et/ou d'oxyde de thorium.

5. Procédé selon l'une quelconque des revendications 1 à 4, **caractérisé en ce que** la dissociation catalytique est réalisée jusqu'à une conversion de 10 à 95%.

6. Procédé selon l'une quelconque des revendications 1 à 5, **caractérisé en ce que** la dissociation est réalisée en phase gazeuse.

7. Procédé selon l'une quelconque des revendications 1 à 6, **caractérisé en ce que** la dissociation est réalisée à une température de 100 à 600°C.

8. Procédé selon l'une quelconque des revendications 1 à 7, **caractérisé en ce qu'**on dissocie le 1-méthoxyoctane, le 1-éthoxyoctane, le tert-butylméthyléther, le tert-amylméthyléther, le tert-amyléthyléther ou le tert-amylbutyléther en α-oléfines correspondantes et en alcools correspondants.
